# EUROPEAN PATENT APPLICATION

(11) **EP 4 186 885 A1**
(43) Date of publication of application: **31.05.2023**
(21) Application number: 21315257.2
(22) Date of filing: 30.11.2021
(51) Int. Cl.: C07C 45/50, C07C 47/02

(54) **PROCESS FOR THE HYDROFORMYLATION OF PROPYLENE**

(71) Applicant: UNIVERSITE DE STRASBOURG, 67000 Strasbourg (FR); Centre National de la Recherche Scientifique, 75794 Paris Cedex 16 (FR)
(72) Inventor: Dydio, Pawel, 77694 KEHL (DE); Sigrist, Michel, 67190 MOLLKIRCH (FR)
(74) Representative: IP Trust

(57) **Abstract**

The invention relates to a process for the hydroformylation of propylene with carbon monoxide and hydrogen to selectively form iso-butanal with respect to n-butanal, the process implementing a hydroformylation catalyst in combination with a phosphine, the hydroformylation catalyst comprising a palladium catalyst which comprises the element PdX₂, where X is a halogen element selected from iodine I and bromine Br, the phosphine is of the form the phosphine is of the form P(R)(R')(R"), where R, R' and R" each comprise an alkyl group or an aryl group or an alkoxy group or an amino group.

## Description

### FIELD OF THE INVENTION

The present invention concerns a method of hydroformylation of propylene which selectively produces iso-butanal with respect of n-butanal.

### TECHNOLOGICAL BACKGROUND OF THE INVENTION

Hydroformylation is a synthetic route to produce aldehydes by chemical reaction of an alkene with carbon monoxide and dihydrogen in the presence of a catalyst, typically a metal complex in combination with an alkyl or aryl phosphine. In particular, the hydroformylation of propylene is one of the most important reactions in the chemical industry. The hydroformylation of propylene produces two isomers of butanal, and more particularly n-butanal and iso-butanal, with a selectivity which depends on the reaction conditions and the nature of the catalyst used. In this respect, the catalysts generally used, which comprise Rh and/or Co-based complexes, favor the formation of n-butanal over iso-butanal.

However, the chemical industry is facing an increasing demand for iso-butanal so that a reverse selectivity in favor of iso-butanal is desirable. This objective has given rise to strong developments, particularly in academic institutes. These developments have essentially been focused on the development of phosphine ligands. In particular, and as proposed in the document [1] cited at the end of the description, ligands have been tailored to modify the intrinsic selectivity of Rh-based catalysts.

By way of example, the document [2] cited at the end of the description discloses the use of a tris-Zn-porphyrin-tris-pyridylphosphine ligand in the form of a capsule allowing selective formation of iso-butanal with respect to n-butanal. However, this selectivity is not maintained when the temperature imposed during the hydroformylation of propylene is higher than 70 °C, a temperature required in industrial environments.

The paper [3] cited at the end of the description proposes another type of ligand that also allows the selective formation of iso-butanal with respect to n-butanal.

Nevertheless, the solutions proposed in these documents are not satisfactory.

Indeed, the proposed ligands are both costly and complicated to implement in industrial environment.

It is therefore an object of the present invention to provide a process for the hydroformylation of propylene to selectively form iso-butanal with respect to n-butanal which is compatible with the requirements of industrial production.

### BRIEF DESCRIPTION OF THE INVENTION

The present invention relates to a process for the hydroformylation of propylene with carbon monoxide and hydrogen to selectively form iso-butanal with respect to n-butanal, the process implementing a hydroformylation catalyst combined with a phosphine,
the hydroformylation catalyst comprising a palladium catalyst which comprises the element PdX₂ , where X is a halogen element selected from iodine I and bromine Br ; the phosphine is of the form P(R)(R')(R"), where R, R' and R" each comprise an alkyl group or an aryl group or an alkoxy group or an amino group.

According to on embodiment, the phosphine is selected from one of the following: P(Me)₃, P(Et)₃, P(n-Bu)₃, P(n-Hex)₃, P(i-Bu)₃, P(i-Pr)₃, P(Cy)₃, P(Cyp)₃, P(t-Bu)₃, P(Bn)₃, P(Cy)₂(t-Bu) , P(Cy)(t-Bu)₂, P(Me)(t-Bu)₂, P(i-Pr)₂(t-Bu) , P(1-Ad)₂(n-Bu) , P(Ph)(t-Bu)₂.

According to on embodiment, the molar ratio of halogen element X to palladium is in the 1 to 3 range, advantageously in the 2 to 2.5 range.

According to on embodiment, the halogen element X is iodine I.

According to on embodiment, the molar ratio of phosphine P(R)(R')(R") to palladium is in the 1 to 4 range, advantageously in the 2 to 3 range.

According to on embodiment, the phosphine comprises the element P(Cy)₃.

According to on embodiment, the palladium catalyst employs an additional palladium element, advantageously this additional palladium element comprises Pd(OAc)₂.

According to on embodiment, said process is carried out in a non-aqueous solvent which comprises at least one of the elements selected from: anisole, toluene, 1,4-dioxane, dimethylacetamide, p-xylene, decahydronaphthalene, DMF, DMSO, α,α,α-trifluorotoluene, hexafluorobenzene, 1,2-dichlorobenzene, methyl benzoate.

According to on embodiment, said process is carried out at a temperature of between 70 °C and 200 °C, advantageously in the 80 to 120 °C range.

### BRIEF DESCRIPTION OF THE DRAWINGS

Further features and advantages of the invention will be apparent from the following detailed description with reference to the attached figures in which :
Figure 1 shows the chemical reaction associated with the hydroformylation of propylene with carbon monoxide CO and dihydrogen in the presence of a catalyst according to the present invention;
Figure 2 is a graphical representation of the evolution of the selectivity of the hydroformylation reaction in the presence of a phosphine of the form P(Cy)₃, and a catalyst of the type PdX₂, the vertical axis representing the selectivity while the horizontal axis lists the different catalysts employed ;
Figure 3 is a graphical representation of the evolution of the catalytic activity in the presence of an alkyl phosphine of the form P(Cy)₃, and a catalyst of the type PdX₂, the vertical axis representing the catalytic activity while the horizontal axis lists the different catalysts employed;
Figure 4 shows the evolution of the catalytic activity as a function of the L/Pd ratio (vertical axis, "L" being a phosphine ligand) and the I⁻/Pd ratio (horizontal axis);
Figure 5 shows the evolution of the selectivity S as a function of the L/Pd ratio (vertical axis) and the I⁻/Pd ratio (horizontal axis).

### DETAILED DESCRIPTION OF THE INVENTION

The present invention relates to a process for the hydroformylation of propylene with carbon monoxide and hydrogen to selectively form iso-butanal (denoted (b)) with respect to n-butanal (denoted (1)). Moreover, the selectivity S of the hydroformylation reaction is defined in the context of the present invention as the ratio n_{b} to nₗ, where n_{b} and nₗ are the respective quantities (for example in moles) of iso-butanal and n-butanal obtained during the hydroformylation of propylene.

The process according to the terms of the present invention uses, in particular, a catalyst, called hydroformylation catalyst, combined with a phosphine. More particularly, the hydroformylation catalyst comprises the element PdX₂, where X is a halogen element chosen from iodine I, bromine Br, while the phosphine of formula P(R)(R')(R"), where R, R' and R" each comprise an alkyl group or an aryl group or an alkoxy group or an amino group.

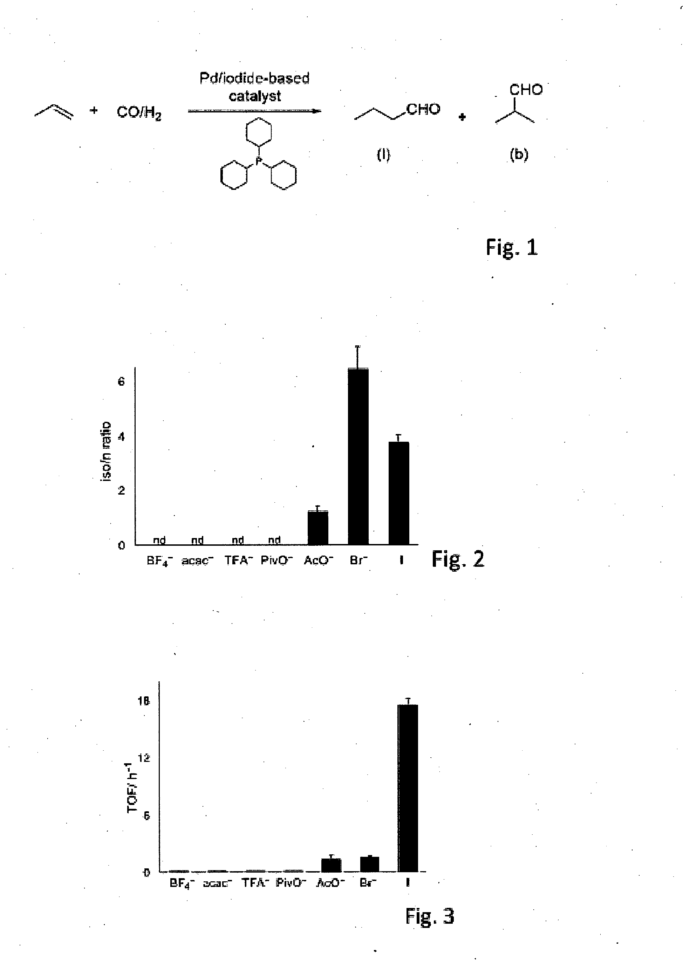

Figure 1 shows the chemical reaction associated with the hydroformylation of propylene with carbon monoxide CO and dihydrogen in the presence of a catalyst according to the present invention.

It is known that such a chemical reaction leads to the formation of iso-butanal and n-butanal in proportions that, depend on the reaction conditions and the nature of the catalyst and/or phosphine employed.

In this respect, in order to promote the formation of iso-butanal with respect to n-butanal, it is proposed in the present invention to use a palladium-based catalyst, and more particularly a catalyst which comprises PdX₂ where X is a halogen. In particu̇lar, X can comprise at least one of the elements selected from: iodine I, bromine Br.

Furthermore, and still in accordance with the terms of the present invention, the phosphine is of the form P(R)(R')(R"), where R, R' and R" each comprise an alkyl group or an aryl group or an alkoxy group or an amino group.

Figure 2 is a graphical representation of the evolution of the selectivity of the hydroformylation reaction of propylene in the presence of a phosphine of the form P(Cy)₃, (Cy : cyclohexyl) and a catalyst of the type PdX₂.

Notably, in this figure 2, the element X may comprise at least one of the anions selected from : BF 4⁻, acac⁻ (acetylacetone ion), TFA⁻ (Tetrafluoroacetic ion), PivO⁻ (pivalic ion), AcO⁻(acetate ion), Br ⁻, I ⁻.

It can be clearly observed on the graph in figures 2 and 3 that catalysts bearing the ions BF₄⁻, acac⁻, TFA⁻, or PivO⁻, do not favor the formation of either iso-butanal or n-butanal. In turn, the catalyst bearing AcO⁻ favors the formation of a mixture of both products in a close to equimolar ratio. Conversely, catalysts bearing Br⁻ and I⁻ ions allow the selective formation of iso-butanal with respect to n-butanal.

More particularly, the consideration of Br⁻ or I⁻ ions increase the selectivity S above 4.

Figure 3 illustrates the catalytic activity (TOF/h^{-l}) for each of the ions listed with reference to Figure 2, and in the case of a phosphine of the form P(Cy)₃.

This figure also demonstrates very clearly that Br⁻ ions, as well as the I⁻ ion, allows a catalytic activity of at least 2 h⁻¹ to be achieved and equal to 17 h⁻¹ in the case of the I⁻ ion.

The hydroformylation reaction shown in Figure 1 is advantageously carried out at a temperature between 70 °C and 200 °C, more particularly between 80 °C and 120 °C.

These temperatures ensure sufficient activity of the catalyst, and more particularly reaction kinetics compatible with the requirements of industrial production.

Advantageously, the hydroformylation reaction is carried out in the presence of ligand selected from the following phosphines : P(Me)₃, P(Et)₃, P(n-Bu)₃, P(n-Hex)₃, P(i-Bu)₃, P(i-Pr)₃, P(Cy)₃, P(Cyp)₃, P(t-Bu)₃, P(Bn)₃, P(Cy)₂(t-Bu), P(Cy)(t-Bu)₂, P(Me)(t-Bu)₂, P(i-Pr)₂(tBu), P(1-Ad)₂(n-Bu), P(Ph)(t-Bu)₂.

In this respect, the following table 1 shows the catalytic activity (TOF) as a function of the different phosphines of formula P(R)(R')(R") mentioned above.

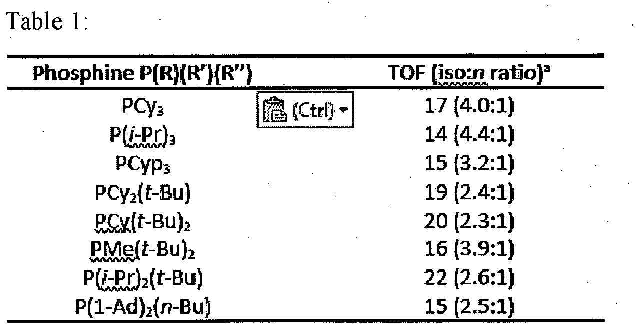

The experimental conditions used to obtain these catalytic activities are as follows:
- Catalyst considered: 10 µmol PdI₂;
- solvent considered: 2 mL anisole;
- pressure of propylene: 4 bar;
- pressure of carbon monoxide: 20 bar;
- pressure of dihydrogen: 80 bar;
- volume of solvent: 2 mL;
- imposed temperature: 100°C

It is thus notable that each of the solvents considered ensures sufficient activity of the catalyst, and more particularly reaction kinetics compatible with the requirements of industrial production. Phosphines cited provide an activity among of 14 h⁻¹ and 22 h⁻¹.

This table also lists the selectivity S (iso:n ratio) obtained in the presence of each of these phosphines of formula P(R)(R')(R").

Advantageously, the hydroformylation reaction is carried out in the presence of a solvent selected from the following solvents: anisole, toluene, 1,4-dioxane, dimethylacetamide, p-xylene, decahydronaphthalene, DMF (N,N-dimethylformamide), DMSO (dimethylsulphoxide), α,α,α-trifluorotoluene,hexafluorobenzene, 1,2-dichlorobenzene, methyl benzate.

In this respect, the following table 2 shows the catalytic activity (TOF) as a function of the different solvents mentioned above.

**Table 2:**

| **Solvent** | **TOF (iso:*n* ratio)** |
|---|---|
| anisole | 17 (4.0:1) |
| toluene | 7 (3.8:1) |
| 1,4-dioxane | 7 (4.4:1) |
| dimethylacetamide | 7 (4.6:1) |
| *p*-xylene | 6 (2.9:1) |
| decahydronaphthalene | 3 (2.1:1) |
| DMF | 5 (2.4:1) |
| DMSO | 1 (5.2:1) |
| CF₃Toluene | 8 (3.4:1) |
| Hexafluorobenzene | 4 (2.9:1) |
| 1,2-dichlorobenzene | 13 (3.2:1) |
| Methyl benzoate | 9 (3.8:1) |

The experimental conditions used to obtain these catalytic activities are as follows:
- Catalyst considered: 10 µmol PdI₂;
- ligand considered: 20 µmol PCy₃;
- pressure of propylene: 4 bar;
- pressure of carbon monoxide: 20 bar;
- pressure of dihydrogen: 80 bar;
- volume of solvent: 2 mL;
- imposed temperature: 100°C.

It is thus notable that each of the solvents considered ensures sufficient activity of the catalyst, and more particularly reaction kinetics compatible with the requirements of industrial production.

Anisol and 1,2-dichlorobenzene provide an activity of 17 h⁻¹ and 13 h⁻¹ respectively.

This table also lists the selectivity S (iso:n ratio) obtained in the presence of each of these solvents. The latter, whatever the solvent considered, remains favorable to the formation of iso-butanal compared to n-butanal.

According to the present invention, it is also possible to consider different molar ratios X to Pd. In particular, the molar ratios X to Pd can be adjusted by considering a catalyst formed by a mixture of Pd(OAc)₂, PdX₂, PPh₄X (or other sources of Pd or X).

In particular, Tables 3, 4 and 5 group the experimental results in terms of selectivity S and catalytic activity (TOF) for the propylene hydroformylation reaction for different experimental conditions.

In each of these cases, the hydroformylation reaction is carried out under the following conditions:
- pressure of the propylene: 4 bar;
- pressure of carbon monoxide: 20 bar;
- pressure of dihydrogen: 80 bar;
- solvent: 2 mL anisole;
- imposed temperature: 100°C.

Thus, Tables 3 to 5 show the evolution of the selectivity S and the catalytic activity TOF as a function of the respective proportions (noted "I⁻/Pd") of the compounds Pd(OAc)₂, PdX₂, PPh₄X, where X corresponds to the anion I⁻.

In particular, for each of these tables:
- the column "Cat. + iodide additive (µmol)" indicates the quantities in µmol (in brackets) of each of the elements Pd(OAc)₂, PdI₂, PPh₄I. ;
- the column "PCy₃ (µmοl)" indicates the amount in µmol of PCy₃;
- the column "Pd/L" indicates the molar ratio of the elements Pd to PCy₃ considered.

**Table 3:**

| **I⁻/Pd** | **Pd/L** | **Cat. + iodide additive (µmοl)** | **PCy₃ (µmοl)** | **TOF** | **S** |
|---|---|---|---|---|---|
| 0 | | Pd(OAc)₂ (10) | | 1,40 | 1,23 |
| 0,5 | | Pd(OAc)₂ (7.5) + Pdl₂ | | 10,90 | 1,65 |
| | | (2.5) | | | |
| 1 | | Pd(OAc)₂ (5) + Pdl₂ (5) | | 18,83 | 3,69 |
| 1,5 | 1/2 | Pd(OAc)₂ (2.5) + Pdl₂ | 20 | 17,25 | 3,39 |
| | | (7.5) | | | |
| 2 | | Pdl₂ (10) | | 16,92 | 3,98 |
| 2,5 | | Pdl₂ (10) + PPh₄l (5) | | 12,32 | 4,43 |
| 3 | | Pdl₂ (10) + PPh₄l (10) | | 13,29 | 3,24 |

**Table 4:**

| **I⁻/Pd** | **Pd/L** | **Cat. + iodide additive (µmοl)** | **PCy₃ (µmol)** | **TOF** | **S** |
|---|---|---|---|---|---|
| 0 | | Pd(OAc)₂ (10) | | 1,93 | 1,69 |
| 0,5 | | Pd(OAc)₂ (7.5) + Pdl₂ | | | |
| | | (2.5) | | 11,55 | 3,33 |
| 1 | | Pd(OAc)₂ (5) + Pdl₂ (5) | | 9,28 | 3,65 |
| 1,5 | 1/1 | Pd(OAc)₂ (2.5) + Pdl₂ | 10 | | |
| | | (7.5) | | 8,93 | 3,54 |
| 2 | | Pdl₂ (10) | | 6,47 | 4,29 |
| 2,5 | | Pdl₂ (10) + PPh₄l (5) | | 6,65 | 4,54 |
| 3 | | Pdl₂ (10) + PPh₄l (10) | | 9,22 | 2,55 |

**Table 5:**

| **I⁻/Pd** | **Pd/L** | **Cat. + iodide additive (µmοl)** | **PCy₃ (µmol)** | **TOF** | **S** |
|---|---|---|---|---|---|
| 0 | | Pd(OAc)₂ (10) | | 0,81 | 1,91 |
| 0,5 | | Pd(OAc)₂ (7.5) + Pdl₂ | | | |
| | | (2.5) | | 5,77 | 3,10 |
| 1 | | Pd(OAc)₂ (5) + Pdl₂ (5) | | 4,72 | 3,28 |
| 1,5 | 2/1 | Pd(OAc)₂ (2.5) + Pdl₂ | 5 | | |
| | | (7.5) | | 3,60 | 3,39 |
| 2 | | Pdl₂ (10) | | 2,64 | 4,78 |
| 2,5 | | Pdl₂ (10) + PPh₄l (5) | | 3,23 | 4,79 |
| 3 | | Pdl₂ (10) + PPh₄l (10) | | 3,94 | 2,26 |

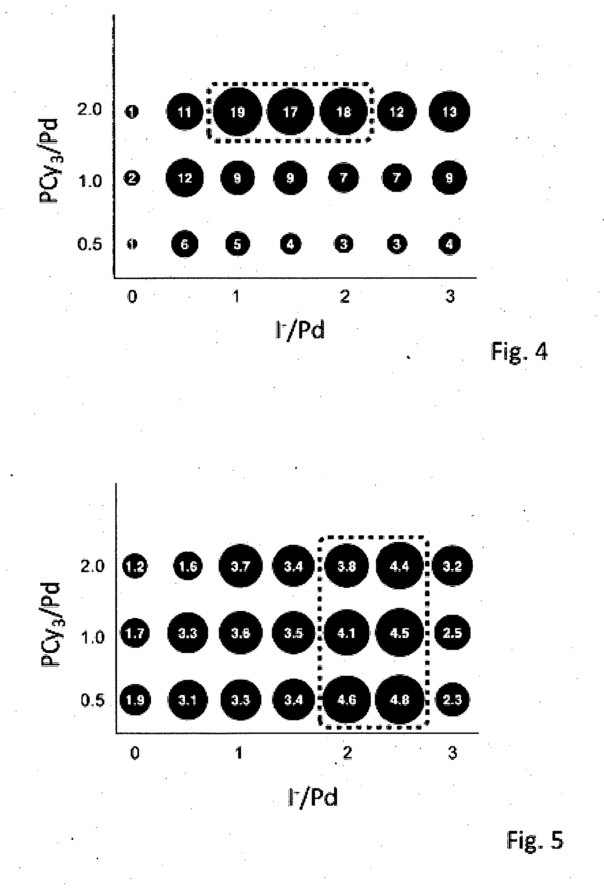

Figure 4 and Figure 5 show the results of table 3 to 5 in graphical form.

In particular, Figure 4 graphically represents the evolution of the catalytic activity as a function of the L/Pd ratio (vertical axis) and the I⁻/Pd ratio (horizontal axis), while Figure 5 graphically represents the evolution of the selectivity S as a function of the L/Pd ratio (vertical axis) and the I⁻/Pd ratio (horizontal axis).

Thus, in accordance with the results illustrated in Figure 5, a molar ratio of the halogen element X to palladium of between 1 and 3, advantageously between 2 and 2.5, makes it possible to achieve a selectivity S of more than 3 or even more than 4.

Equivalently, the results illustrated in Figure 5, with a molar ratio of phosphine P(R)(R′)(Rʺ) to palladium of between 1 and 4, advantageously between 2 and 3, makes it possible to achieve a catalytic activity greater than 7, or even greater than 15.

Of course, the. invention is not limited to the embodiments described and alternative embodiments may be made without departing from the scope of the invention as defined by the claims.

### REFERENCES

[1] Ning, Y. et al, "Transition Metal-Catalyzed Branch-Selective Hydroformylation of Olefins in Organic Synthesis" Green Synth. Catal. 2021, S2666554921000399 ;
[2] US8710275B2 ;
[3] Iu, L. et al, "High Iso Aldehyde Selectivity in the Hydroformylation of Short-Chain Alkenes", Angew. Chem. Int. Ed. 2019, 58 (7), 2120-2124.

## Claims

1. A method for the hydroformylation of propylene with carbon monoxide and hydrogen to selectively form iso-butanal with respect to n-butanal, the process implementing a hydroformylation catalyst combined with a phosphine,
the hydroformylation catalyst comprising a palladium catalyst which comprises the element PdX₂ , where X is a halogen element selected from iodine I and bromine Br, the phosphine is of the form P(R)(R')(R"), where R, R′ and Rʺ each comprise an alkyl group or an aryl group or an alkoxy group or an amino group.

2. The method of claim 1, wherein the phosphine is selected from one of the following: P(Me)₃, P(Et)₃, P(n-Bu)₃, P(n-Hex)₃, P(i-Bu)₃, P(i-Pr)₃, P(Cy)₃, P(Cyp)₃, P(t-Bu)₃, P(Bn)₃, P(Cy)₂(t-Bu), P(Cy)(t-Bu)₂, P(Me)(t-Bu)₂, P(i-Pr)₂(t-Bu), P(1-Ad)₂(n-Bu), P(Ph)(t-Bu)₂.

3. The method according to claim 1 or 2, wherein the molar ratio of halogen element X to palladium is in the 1 to 3 range, advantageously in the 2 to 2.5 range.

4. The method according to claim 3, wherein the halogen element X is iodine I.

5. The method according to claim 3, wherein the halogen element X is bromine Br.

6. The method according to any of claims 1 to 5, wherein the molar ratio of phosphine P(R)(R')(R") to palladium is in the 1 to 4 range, advantageously in the 2 to 3 range.

7. The method of claim 4 to 6, wherein the phosphine comprises the element P(Cy)₃.

8. The method according to any of claims 1 to 7, wherein the palladium catalyst employs an additional palladium element, advantageously this additional palladium element comprises Pd(OAc)₂.

9. The method according to any one of claims 1 to 8, wherein said process is carried out in a non-aqueous solvent which comprises at least one of the element selected from: anisole, toluene, 1,4-dioxane, dimethylacetamide, p-xylene, decahydronaphthalene, DMF, DMSO, α,α,α-trifluorotoluene,hexafluorobenzene, 1,2-dichlorobenzene, methyl benzoate.

10. The method according to any of claims 1 to 9, wherein said process is carried out at a temperature of between 70 °C and 200 °C, advantageously in the 80 to 120 °C range.
